(19) Europäisches Patentamt
European Patent Office
Office européen des brevets

(11) Veröffentlichungsnummer: **0 212 536**
**B1**

(12) # EUROPÄISCHE PATENTSCHRIFT

(45) Veröffentlichungstag der Patentschrift: **10.10.90**

(51) Int. Cl.$^5$: **C 07 H 19/14**

(21) Anmeldenummer: **86111117.7**

(22) Anmeldetag: **12.08.86**

(54) **7-Desaza-2'-desoxyguanosin-nukleotide, Verfahren zu deren Herstellung und deren Verwendung zur Nukleinsäure-Sequenzierung.**

(30) Priorität: **16.08.85 DE 3529478**

(43) Veröffentlichungstag der Anmeldung:
**04.03.87 Patentblatt 87/10**

(45) Bekanntmachung des Hinweises auf die Patenterteilung:
**10.10.90 Patentblatt 90/41**

(84) Benannte Vertragsstaaten:
**AT BE CH DE FR GB IT LI LU NL SE**

(56) Entgegenhaltungen:
**CHEMICAL ABSTRACTS, Band 105, 1986, Seite 768, Zusammenfassung 191556e, Columbus, Ohio, US; F. SEELA et al.: "Self-complementary oligomers containing 7-deaza-2'-deoxyguanosine or 2'-deoxytubercidin", & CHEM. SCR. 1986, 26(1), 173-8**

(73) Patentinhaber: **BOEHRINGER MANNHEIM GMBH**
**Sandhofer Strasse 116**
**D-6800 Mannheim 31 (DE)**

(72) Erfinder: **Seela, Frank, Prof.**
**Dahler Heide 87**
**D-4790 Paderborn (DE)**

(56) Entgegenhaltungen:
**CHEMICAL ABSTRACTS, Band 105, 1986, Seite 396, Zusammenfassung 222147d, Columbus, Ohio, US; P.J. BARR et al.: "7-Deaza-2'-deoxyguanosine-5'-triphosphate: enhanced resolution in M13 dideoxy sequencing", & BIOTECHNIQUES 1986,4(5), 428-32**

**NUCLEIC ACIDS RESEARCH, Band 14, Nr. 3, 1986, Seiten 1319-1324, IRL Press LTD, Oxford, GB; S. MIZUSAWA et al.: "Improvement of the dideoxy chain termination method of DNA sequencing by use of deoxy-7-deazaguanosine triphosphate in place of dGTP"**

**EP 0 212 536 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft 7-Desaza-2'-desoxyguanosin-nukleotide, ein Verfahren, zu deren Herstellung dieser Verbindungen aus 7-Desaza-2'-desoxyguanosin sowie die Verwendung der Nukeotide zur Sequenzierung von Nukleinsäuren.

Neben der chemischen Methode zur Sequenzierung von DNA nach Maxam und Gilbert wird zur Aufklärung unbekannter DNA-Sequenzen vor allem die enzymatische "Plus-Minus"-Methode nach Sanger und Coulson häufig angewendet. Diese Verfahren sind der Fachwelt geläufig.

Als Alternative zur "Plus-Minus"-Methode nach Sanger und Coulson, J. Mol. Biol. *94,* 441—448 (1975), wurde zur Sequenzierung von Desoxyribonucleinsäure (DNA) von Sanger, Nicklen und Coulson, Proc. Nat. Acad. Sci. USA *74,* 5463—5467 (1977), ein Verfahren entwickelt, welches auf der Verwendung DNA-Polymerase inhibierender Nukleosidanaloga basiert. Da hierfür neben Arabinonukleotiden vor allem 2',3'-Didesoxynukleotide eingesetzt werden, wird diese Methode auch als Didesoxy-Methode bezeichnet.

2',3'-Didesoxynukleosid-triphosphate werden in Gegenwart von DNA-Polymerase in wachsende Oligonukleotid-Ketten an Stelle der "richtigen" Nukleosid-Triphosphate eingebaut. Da sie jedoch keine 3'-Hydroxygruppe besitzen, kann die Kette dort nicht weiter verlängert werden. Das Kettenwachstum endet überall dort, wo ein 2',3'-Didesoxynukleosid-triphosphat eingebaut wird.

Dieser Effekt wird zur Sequenzierung von DNA ausgenutzt, indem die zu untersuchende Einzelstrang-DNA in vier Proben geteilt wird. Jede dieser Proben wird mit einem kurzen, eventuell radioaktiv markierten DNA-Statermolekül, mit DNA-Polymerase, mit 3 verschiedenen Desoxyribonukleosid-triphosphaten, wovon eines radioaktiv markiert sein kann und mit einer Mischung des jeweils noch fehlenden, in jedem der vier Ansätze unterschiedlichen, vierten Nukleosid-triphosphats mit dem entsprechenden 2',3'-Didesoxy-Nukleotidanalogon, inkubiert. Nachdem das kurze Startfragment an den Nukleinsäurestrang anhybridisiert ist, beginnt die Polymerase an der 3'-OH-Gruppe des Startermoleküls mit der Synthese des zur zu untersuchenden DNA komplementären Moleküls. Das Enzym verlängert dieses Molekül so lange, bis ein 2',3'-Didesoxynukleotid eingebaut worden ist. Nach Beendigung der Reaktion und Abspaltung des als Matrize dienenden DNA-Stranges durch Denaturierung liegt deshalb eine Mischung von Fragmenten vor, die in allen vier Ansätzen das gleiche 5'-Ende besitzen und die in jedem der vier Ansätze das jeweilige, verwendete Didesoxyanalogon als 3'-Ende aufweisen. Die gelelektrophoretische Auftrennung dieser Moleküle ergibt für jeden Ansatz ein Bandenmuster, welches die Verteilung des dem jeweiligen Nukleosidanalogon entsprechenden Nukleotids in der neu synthetisierten DNA widergibt. Durch Vergleich der Bandenmuster der vier verschiedenen Ansätze, die nebeneinander auf einem Gel aufgetrennt werden, kann direkt die Sequenz der neusynthetisierten DNA und aufgrund der Kenntnis der basenspezifischen Paarung auch die Sequenz der als Matrize dienenden, zu untersuchenden Nukleinsäure abgelesen werden.

Vorteile der Didesoxy-Methode sind vor allem in ihrer einfachen Durchführbarkeit und der Vermeidung eines zusätzlichen, bei der "Plus-Minus"-Methode erforderlichen, Inkubations- und Reinigungsschrittes zu sehen.

Probleme treten bei Sequenzierungen von Nukleinsäuren nach der Didesoxy-Methode, jedoch auch bei anderen Sequenzierungsmethoden, die DNA-Polymerase verwenden, bei Cytosin-Guanin-reichen Regionen auf. In Einzelstrang-DNA bilden Cytosin-Guanin-reiche Sequenzen durch interne Basenpaarung stabile Schleifen. Dies wirkt sich bei der gelelektrophoretischen Trennung solcher Fragmente in einer mangelhaften Auflösung aus. Guaninnukeotide sind außerdem nur mäßig stabil und tendieren in wässriger Lösung dazu, Aggregate zu bilden. Dies führt zu Schwierigkeiten bei der enzymatischen Polymerisation, da an der aktiven Stelle des Enzyms nicht genug Substrat verfügbar ist. Als Folge davon ist es oft schwierig, Cytosin-Guanin-reiche Regionen in Nukleinsäuren nach der Didesoxy-Methode richtig zu sequenzieren.

Aufgabe war es deshalb, Guanosin-nukleotidanaloga zu finden, welche stabil sind, keine Selbstaggregation eingehen und stellvertretend für 2'-Desoxyguanosin-nukleotide als Substrat für DNA-Polymerase, z.B. bei Sequenzierungen von Nukleinsäuren nach der Didesoxy-Methode verwendet werden können.

Gelöst wird diese Aufgabe durch die erfindungsgemäßen 7-Desaza-2'-desoxyguanosin-nucleotide der allgemeinen Formel I

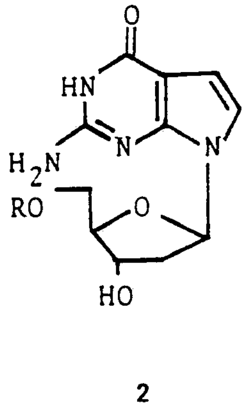

(I)

in der R $—PO_3H_2$, $—P_2O_6H_3$, $—P_3O_9H_4$ oder ein Alkali, Erdalkali- oder Ammoniumsalz der genannten Säurereste bedeutet.

Unter Alkalisalzen der in der Definition von R genannten Säurereste sind vor allem Lithium-, Natrium- und Kaliumsalze zu verstehen.

Erdalkalisalze der in der Definition von R genannten Säurereste sind vor allem Magnesium- oder Calciumsalze.

Ammoniumsalze der in der Definition von R genannten Säurereste können unsubstituierte Ammoniumionen enthalten oder solche, die durch Alkylgruppen mit 1—7, bevorzugt 1—4 Kohlenstoffatomen substituiert sind. Ganz besonders vorteilhaft sind Triethyl- und Tri-butylammnoniumionen. Substituenten der Ammoniumionen können jedoch auch Aralkylreste sein. Bevorzugt ist hier der Benzylrest. Die Substituenten eines Ammoniumions können sowhol gleich als auch verschieden sein.

Die erfindungsgemäßen Verbindungen der allgemeinen Formel I sind neu. Sie neigen nicht zu den für 2'-Desoxy- oder 2',3'-Didesoxyguanosin-Derivate typischen Aggregationen. Das Triphosphat der allgemeinen Formel I wird außerdem statt 2'-Desoxyguanosintriphosphat von DNA-Polymerase in wachsende DNA-Ketten eingebaut.

Hergestellt werden Verbindungen der allgemeinen Formel I nach an sich bekannten Methoden aus 7-Desaza-2'-desoxyguanosin, welches nach H.-D. Winkeler & F. Seela, J. Org. Chem. *48,* 3119—3122 (1983) darstellbar ist.

Durch Umsetzung von 7-Desaza-2'-desoxyguanosin mit einem Phosphorylierungsmittel, wie z.B. Phosphoroxychlorid in einem Trialkylphosphat, vorzugsweise, Trimethylphosphat, als Lösungsmittel und anschließende geeignete Aufarbeitung ist das 5'-Monophosphat der Formel Ia

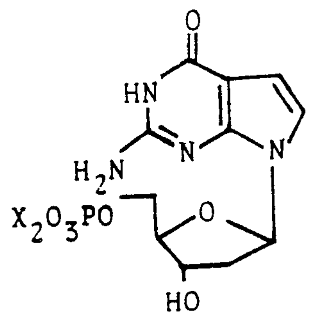

in der X Wasserstoff, ein Alkali-, Erdalkali- oder Ammoniumion bedeutet, zugänglich.

Alkali-, Erdalkali- und Ammoniumionen haben in der Definition von X die gleiche Bedeutung wie in der Definition von R.

Vorzugsweise wird die Phosphorylierung bei niedriger Temperatur, besonders vorteilhaft bei 0—10°C, durchgeführt. Die Reaktionszeit beträgt 5 Stunden bis 1 Tag, bevorzugt 7—15 Stunden.

Die Aufarbeitung erfolgt beispielsweise durch Hydrolyse der Reaktionsmischung mit Eis, anschließende Neutralisation und Isolierung des Produkts mittels Ionenaustauschchromatographie.

Die Position der Phosphorylierung kann durch Proton-entkoppelte $^{13}C$-NMR-Spektroskopie ermittelt werden. Als Indikator der 0-5'-Phosphorylierung dient die $^3J$(P-C) Kopplungskonstante von C-4'. Sie beträgt 8 Hz.

5'-Di- bzw. 5'-Triphosphate der allgemeinen Formel I werden hergestellt, indem ein Trialkylammoniumsalz des 5'-Monophosphats der Formel Ia aktiviert und anschließend mit einem Trialkylammoniumphosphat bzw. -diphosphat kondensiert wird. Als Trialkylammoniumionen werden bevorzugt Tributylammoniumionen eingesetzt. Die Aktivierung erfolgt vorteilhafterweise unter wasserfreien Bedingungen mittels 1,1'-Carbonyldiimidazol bei Raumtemperatur in einem polaren, aprotischen Lösungsmittel, wie z.B. Dimethylformamid. Die Kondensation wird ebenfalls bei Raumtemperatur in einem polaren, aprotischen Lösungsmittel durchgeführt. Bevorzugt ist Dimethylformamid. Die Reaktionszeiten für Aktivierung und Kondensationsreaktion betragen jeweils 3 Stunden bis 3 Tage.

Der Erfolg der Phosphorylierung dann durch $^{31}P$-NMR-Spektroskopie kontrolliert werden. Es wird hier auf Tabelle 1 verwiesen, in der die chemische Verschiebung der Phosphorsignale der Triethylammoniumsalze von 7-Desaza-2'-desoxyguanosin-mono-, -di- und -triphosphat aufgelistet sind.

Ein weiterer Gegenstand der Erfindung ist die Verwendung von Verbindungen der allgemeinen Formel I bei der Sequenzierung von Nukleinsäuren.

7-Desaza-2'-desoxyguanosin-triphosphat kann anstelle von 2'-Desoxyguanosin-triphosphat bei solchen Sequenzierungsmethoden für DNA verwendet werden, bei denen der Einsatz von DNA-Polymerase erforderlich ist. Besonders bevorzugt ist jedoch die Verwendung bei der Didesoxy-Sequenzierungsmethode nach Sanger (Proc. Nat. Acad. Sci. USA *74,* 5463—5467 (1977). Hier kann unter Beibehaltung aller weiterer üblichen Bedingungen 2'-Desoxyguanosin-triphosphat durch 7-Desaza-2'-desoxyguanosin-triphosphat ersetzt werden.

Statt 7-Desaza-2'-desoxyguanosin-triphosphat kann auch das entsprechende 5'-Mono- oder 5'-Diphosphat verwendet werden, wenn durch entsprechende zusätzliche Enzyme und Substrate

sichergestellt ist, daß diese Nukleotide in der Inkubationslösung in das 5'-Triphosphat überführt werden können. So muß z.B. Monophosphat der Formel Ia mittels Nukleosidmonophosphatkinase und ATP, bzw. das entsprechende Diphosphat mittels Nukleosiddiphosphatkinase und ATP in das Triphosphat ungewandelt werden.

Werden die erfindungsgemäßen 7-Desaza-2'-desoxyguanosin-nukleotide bei solchen Sequenzierungsmethoden verwendet, so sind keine Störungen durch Sekundärwechselwirkungen zwischen Cytosin und Guanin zu beobachten. Dies führt zu einer wesentlich besseren gelektrophoretischen Trennung Guanin-Cytosin-reicher Sequenzfragmente.

Mit den erfindingsgemäßen Verbindungen ist somit eine störungsfreie Sequenzierung von Cytosin-Guanin-reichen Nukleinsäuren möglich.

Die Erfindung wird durch die folgenden Beispiele näher erläutert.

Beispiel 1

2-Amino-7-(2'-desoxy-β-D-erythro-pentofuranosyl)-3,7-dihydro-4H-pyrrolo-[2,3-d]pyrimidin-4-on-5'-monosphosphat, Triethylammoniumsalz

2-Amino-7-(2'-desoxy-β-D-erythro-pentafuranosyl)-3,7-dihydro-4H-pyrrolo-[2,3-d]pyrimidin-4-on (100 mg, 375 μmol) wurden bei 4°C 10 Stunden lang in Trimethylphosphat (1,5 ml) mit Phosphoroxychlorid (75 μl, 827 μmol) behandelt. Anschließend wurde die Mischung mit Eis hydrolisiert, mit 1 M wässriger Triethylammoniumbicarbonatlösung neutralisiert und danach das Lösungsmittel im Vakuum entfernt. Der Rückstand wurde dann in Wasser gelöst, auf eine 40 × 2,5 cm Ionenaustauschersäule (DEAE-Sephadex®, Hydrogencarbonat-Form) aufgetragen, mit 500 ml Wasser gewaschen und mit einem linearen Gradienten von 0,5 M Triethylammoniumbicarbonat (1000 ml) und Wasser (1000 ml) chromatographiert. Die Hauptzone wurde bei einer Salzkonzentration von 0,45 M eluiert. Die entsprechenden Fraktionen wurden gesammelt und zur Trockne eingeengt. Durch Wiederaufnahme mit Wasser und erneutes Einengen bis zur Trockne wurden die flüchtigen Salze entfernt. Man erhielt so amorphes Produkt (2820 $A_{259}$ Einheiten, 211 μmol) in 56%iger Ausbeute.

UV (Wasser) $\lambda_{max}$ 259 nm (ε 13400)

Phosphatbestimmung: 0,93 mol Phosphat/mol Aglykon

$^{13}$C-NMR ($D_2O$) δ = 8,7 ($CH_3$), 38,3 (C-2'), 64,8 (C-5', breit), 72,2 (C-3'), 83,4 (C-1'), 85,9 (d, J = 8 Hz, C-4')

Beispiel 2

2-Amino-7-(2'-desoxy-β-D-erythro-pentofuranosyl)-3,7-dihydro-4H-pyrrolo-[2,3-d]pyrimidin-4-on-5'-diphosphat, Triethylammoniumsalz

Die in Beispiel 1 hergestellte Verbindung (1500 $A_{259}$ Einheiten, 112 μmol) wurde 30 ml Wasser gelöst und auf eine 25 × 2 cm Kationenaustauschersäule (Merck, Typ 1, Pyridinium-Form) aufgebracht und mit 500 ml Wasser eluiert. Das Eluat wurde zur Trockne eingeengt und mit Tributylamin (27 μl, 112 μmol) versetzt. Wasser wurde durch widerholtes Aufnehmen mit wasserfreiem Dimethylformamid und anschließendes Einengen bis zur Trockne entfernt. Das so hergestellte Tributylammoniumsalz der in Beispiel 1 hergestellten Vebindung wurde dann in wasserfreiem Dimethylformamide (2 ml) gelöst und mit 1,1'-Carbonyl-diimidazol (90 mg, 560 μmol) behandelt. Die Reaktionsmischung wurde 5 Stunden lang bei Raumtemperatur gerührt Überschüssiges 1,1'-Carbonyl-diimidazol wurde dann mit Methanol (35 μl) zerstört. Nach 30 Minuten wurde Tributylammoniumphosphat (560 μmol) in 6 ml Dimethylformamide zugegeben und die Reaktionsmischung 24 Stunden lang bei Raumtemperatur gerührt. Nach Entfernung des Lösungsmittels im Hochvakuum wurde der Rückstand in Wasser gelöst und auf eine 45 × 3,5 cm Ionenaustauschersäule (DE 52 Cellulose, Bicarbonat-Form) aufgetragen. Das Diphosphat wurde mit einem linearen Gradienten von Wasser und 0,5 M Triethylammoniumbicarbonat (jeweils 1000 ml) bei etwa 0,3 M Salzkonzentration eluiert. Entfernung des Lösungsmittels und anschließende Lyophilisation lieferte farbloses, amorphes Produkt (950 $A_{259}$ Einheiten) in 64%iger Ausbeute.

UV (Wasser) $\lambda_{max}$ 259 nm (ε 13400)

Beispiel 3

2-Amino-7-(2'-desoxy-β-D-erythropentofuranosyl)-3,7-dihydro-4H-pyrrolo-[2,3-d]pyrimidin-4-on-5'-triphosphat, Triethylammoniumsalz

Die in Beispiel 1 hergestellte Verbindung (1340 $A_{259}$ Einheiten, 100 μmol) wurden in 10 ml Wasser gelöst, auf eine 25 × 2 cm Kationenaustauschersäule (Merck, Typ 1, Pyridinium-Form) aufgetragen und die Säule mit 500 ml Wasser gewaschen. Das Eluat wurde zur Trockne eingeengt und mit Tributylamin (24 μl, 100 μmol) versetzt. Wasser wurde durch wiederholte Aufnahme mit wasserfreiem Dimethylformamid und anschließendes Einengen bis zur Trockne entfernt. Der Rückstand wurde dann in 2 ml Dimethylformamid gelöst und mit 1,1'-Carbonyldiimidazol (80 mg, 500 μmol), welches in 2 ml Dimethylformamid gelöst war, versetzt. Die Reaktionsmischung wurde 24 Stunden lang bei Raumtemperatur gerührt. Überschüssiges 1,1'-Carbonyldiimidazol wurde mit Methanol (35 μl) zerstört. Anschließend wurde mit Bis-Triethylammoniumpyrophosphat (500 μmol), gelöst in 2 ml Dimethylformamid, bei Raumtemperatur behandelt. Nach 2tägigem Rühren wurde die Reaktionsmischung im Vakuum zur Trockne eingeengt. Der Rückstand wurde in Wasser gelöst und auf eine 40 × 2,5 cm Anionenaustauschersäule (DEAE-Sephadex®, Bicarbonat-Form) aufgetragen. Elution mit einem linearen Gradienten von 0,5 M Triethylammoniumcarbonat und

Wasser (jeweils 1000 ml) ergab bei einer Salzkonzentration von 0,4 M nach Einengung bis zur Trockne das Triphosphat als farblosen, amorphen Feststoff (817 $A_{259}$ Einheiten) in 61%iger Ausbeute.

UV (Wasser) $\lambda_{max}$ 259 nm ($\varepsilon$ 13400)

TABELLE 1

Chemische Verschiebung der $^{31}$P-NMR-Signale der Triethylammoniumsalze der in Beispiel 1, 2 und 3 hergestellten Verbindungen, gemessen in $H_2O/D_2O$ (3:1), welches 100 mM EDTA enthält

| Verbindung hergestellt in | Pα | Pβ | Pγ |
|---|---|---|---|
| Beispiel 1 | +4,65 (s) | | |
| Beispiel 2 | −9,93 (d, J = 23 Hz) | −5,92 (d, J = 23 Hz) | |
| Beispiel 3 | −10,00 (d, J = 20 Hz) | −21,54 (t, J = 20 Hz) | −8,35 (d, J = 20 Hz) |

**Patentansprüche**

1. 7-Desaza-2'-desoxyguanosin-nucleotide der allgemeinen Formel I

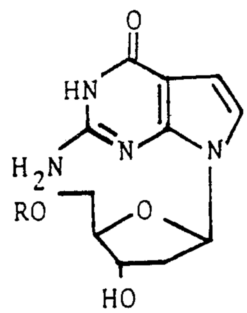

(I)

in der R $-PO_3H_2$, $-P_2O_6H_3$, $-P_3O_9H_4$ oder ein Alkali-, Erdalkali- oder Ammoniumsalz der genannten Säurereste bedeutet.

2. Verfahren zur Herstellung von 7-Desaza-2'-desoxyguanosin-nucleotiden der allgemeinen Formel I

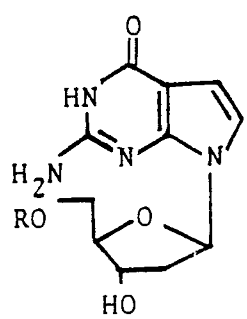

(I)

in der R $-PO_3H_2$, $-P_2O_6H_3$, $-P_3O_9H_4$ oder ein Alkali-, Erdalkali- oder Ammoniumsalz der genannten Säurereste bedeutet, dadurch gekennzeichnet, daß 7-Desaza-2'-desoxyguanosin mit üblichen Phosphorylierungsmitteln in das 5'-Monophosphat der Formel Ia

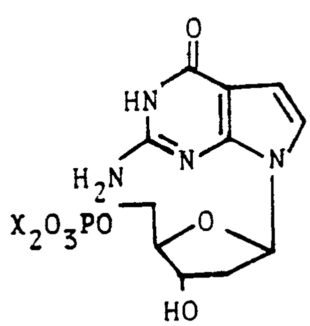

in der X Wasserstoff, ein Alkali- Erdalkali- oder Ammoniumion bedeutet, überführt und das 5'-Monophosphat gegebenenfalls mit einem Ortho- oder Pyrophosphorsäuresalz zum 5'-Di- oder 5'-Triphosphat umgesetzt wird.

3. Verfahren gemäß Anspruch 2, dadurch gekennzeichnet, daß zur Überführung von 7-Desaza-2'-desoxyguanosin in ein 5'-Monophosphat der Formel Ia als Phosphorylierungsmittel Phosphoroxychlorid verwendet wird.

4. Verfahren gemäß Anspruch 2, dadurch gekennzeichnet, daß das 5'-Monophosphat der Formel Ia vor der Umsetzung mit einem Ortho- oder Pyrophosphorsäuresalz in ein aktiviertes Trialkylammoniumsalz überführt wird.

5. Verfahren gemäß Anspruch 4, dadurch gekennzeichnet, daß die Aktivierung des Trialkylammoniumsalzes des 5'-Monophosphats der Formel Ia mittels 1,1'-Carbonyldiimidazol erfolgt.

6. Verwendung von 7-Desaza-2'-desoxyguanosin-nucleotidin gemäß Anspruch 1 als Substrat von DNA-Polymerase.

7. Verwendung von 7-Desaza-2'-desoxyguanosin-nukleotidin gemäß Anspruch 1, anstelle von 2'-Desoxyguanosin-nukleotiden bei der DNA-Polymerase-verwendenden Sequenzierung von Nukleinsäuren.

8. Verwendung gemäß Anspruch 7 bei der Sequenzierung von DNA nach der Didesoxy-Methode von Sanger.

**Revendications**

1. 7-Désaza-2'-désoxyguanosine-nucléotides de formule générale I

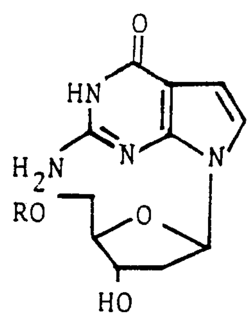

(I)

où R représente $-PO_3H_2$, $-P_2O_6H_3$, $-P_3O_9H_4$ ou un sel de métal alcalin, alcalino-terreux ou d'ammonium du reste acide mentionné.

2. Procédé de préparation de 7-désaza-2'-désoxyguanosine-nucléotides de formule générale I

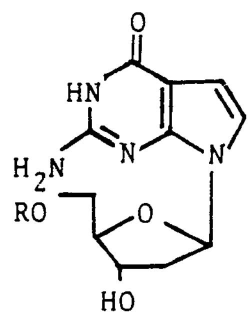

(I)

où R représente $-PO_3H_2$, $-P_2O_6H_3$, $-P_3O_9H_4$ ou un sel de métal alcalin, alcalino-terreux ou d'ammonium du reste acide mentionné, caractérisé en ce que l'on transforme une 7-désaza-2'-désoxyguanosine, à l'aide d'un agent de phosphorylation usuel, en 5'-monophosphate de formule Ia

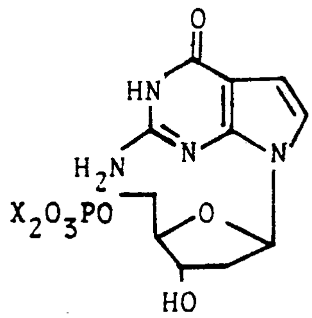

où X représenté l'hydrogène, un ion alcalin, alcalino-terreux ou ammonium, et qu'éventuellement on fair réagir le 5'-monophosphate avec un sel d'acide ortho- ou pyrophosphorique, pour obtenir un 5'-di- ou 5'-triphosphate.

3. Procédé selon la revendication 2, caractérisé en ce que pour transformer la 7-désaza-2'-désoxyguanosine en un 5'-monophosphate de formule Ia, comme agent de phosphorylation on utilise l'oxychlorure de phosphore.

4. Procédé selon la revendication 2, caractérisé en ce qu'avant la réaction, le 5'-monophosphate de formule Ia est transformé, à l'aide d'un sel d'acide ortho- ou pyrophosphorique, en un sel de trialkylammonium activé.

5. Procédé selon la revendication 4, caractérisé en ce que l'activation du sel de trialkylammonium du 5'-monophosphate de formule Ia s'effectue au moyen du 1,1'-carbonyldiimidazole.

6. Utilisation des 7-désaza-2'-désoxyguanosine-nucléotides selon la revendication 1, comme substrat de l'ADN polymérase.

7. Utilisation des 7-désaza-2'-désoxyguanosine-nucléotides selon la revendication 1, à la place des 2'-desoxyguanosine-nucléotides, dans le séquençage des acides nucléiques utilisant l'ADN polymérase.

8. Utilisation selon la revendication 7, dans le séquençage de l'ADN selon la méthode didésoxy de Sanger.

**Claims**

1. 7-Desaza-2'-desoxyguanosine nucleotides of the general formula I

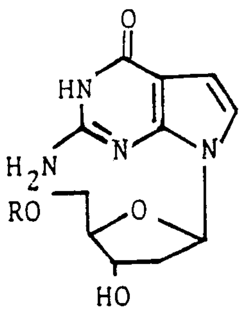

(I)

in which R signifies $—PO_3H_2$, $—P_2O_6H_3$, $—P_3O_9H_4$ or an alkali metal, alkaline earth metal or ammonium salt of the said acid residues.

2. Process for the preparation of 7-desaza-2'-desoxyguanosine nucleotides of the general formula I

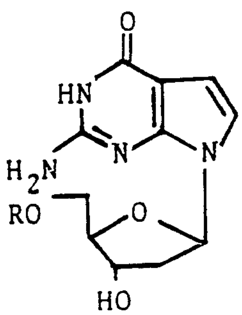

(I)

in which R signifies $—PO_3H_2$, $—P_2O_6H_3$, $—P_3O_9H_4$ or an alkali metal, alkaline earth metal or ammonium salt of the said acid residues, characterized in that 7-desaza-2'-desoxyguanosine is converted with conventional phosphorylation agents into the 5'-monophosphate of the formula Ia

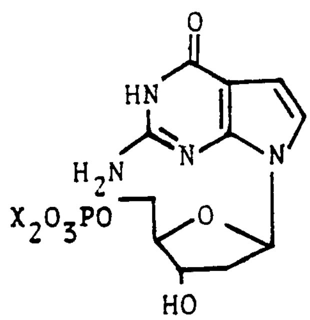

in which X signifies hydrogen, an alkali metal, alkaline earth metal or ammonium ion, and the 5′-monophosphate is optionally reacted with an ortho- or pyrophosphoric acid salt to give the 5′-di- or 5′-triphosphate.

3. Process according to claim 2, characterised in that, for the conversion of 7-desaza-2′-desoxyguanosine into a 5′-monophosphate of the formula Ia, phosphorus oxychloride is used as phosphorylation agent.

4. Process according to claim 2, characterised in that the 5′-monophosphate of the formula Ia is converted into an active trialkylammonium salt before the reaction with an ortho- or pyrophosphoric acid salt.

5. Process according to claim 4, characterised in that the activation of the trialkylammonium salt of the 5′-monophosphate of the formula Ia takes place by means of 1,1′-carbonyldiimidazole.

6. Use of 7-desaza-2′-desoxyguanosine nucleotides according to claim 1 as substrates of DNA polymerase.

7. Use of 7-desaza-2′-desoxyguanosine nucleotides according to claim 1 instead of 2′-desoxyguanosine nucleotides in the sequencing of nucleic acids using DNA polymerase.

8. Use according to claim 7 in the sequencing of DNA according to the didesoxy method of Sanger.